# EUROPEAN PATENT SPECIFICATION

(11) **EP 1 565 758 B1**
(45) Date of publication and mention of the grant of the patent: **10.01.2007**
(21) Application number: 03811966.5
(22) Date of filing: 27.11.2003
(51) Int. Cl.: G01R 33/28, A61B 5/055

(54) **METHOD OF MAGNETIC RESONANCE IMAGING**
VERFAHREN FÜR DIE MAGNETISCHE RESONANZBILDGEBUNG
METHODE D'IMAGERIE PAR RESONANCE MAGNETIQUE

(30) Priority: 27.11.2002 NO 20025711
(43) Date of publication of application: 24.08.2005
(73) Proprietor: GE Healthcare AS, 0401 Oslo (NO)
(72) Inventor: AXELSSON, Oskar, Amersham Health R & D AB, PO Box 4330 Nydalen, N-0401 Oslo (NO); GOLMAN, Klaes, Amersham Health R & D AB, PO Box 4330 Nydalen, N-0401 Oslo (NO); MANSSON, Sven, Amersham Health R & D AB, PO Box 4330 Nydalen, N-0401 Oslo (NO); PETERSSON, Stefan, Amersham Health R & D AB, PO Box 4330 Nydalen, N-0401 Oslo (NO)
(74) Representative: Canning, Lewis R.
(86) International application number: PCT/NO2003/000395
(87) International publication number: WO 2004/048988

(56) References cited:
- WO-A-98/22022
- WO-A-99/35508
- WO-A-02/088766
- US-A- 5 154 179
- US-A- 5 211 166

## Description

### Field of the invention

The invention relates to magnetic resonance imaging (MRI) of invasive devices, e.g. during surgical and therapeutic procedures.

### Description of related art

Interventional procedures in radiology are very common and increasingly popular. They are normally performed under fluoroscopic feedback by insertion of invasive instruments and/or devices like catheters, guide wires, biopsy needles, etc. through the blood vessels. The procedures can last for several hours and involve very high radiation doses for both the patient and the personnel performing them. During these procedures, it is desirable that the physician is able to locate or guide the invasive devices inserted into the patient's body when these devices are no longer visible. Since soft body parts give very little contrast on X-ray, it is often necessary to use high doses of iodinated contrast agents to localize the devices which in turn may cause problems, in particular for patients with reduced kidney function.

MRI-guided interventional procedures have gained increasing importance in recent years. Such MRI-guided procedures can be divided into two categories: intraoperative procedures, which integrate surgery with MRI, and interventional procedures for guiding, monitoring and controlling therapy. Intraoperative procedures generally require an open magnet MR imager and are desirable as the MRI can be used to define anatomy and monitor tissue function as it changes during surgery. Interventional procedures generally require only limited patient access and thus conventional, closed magnet MR imagers may be used. Normally, a MRI contrast agent or contrast medium is employed in such methods which enhances contrast and thus provides images of a higher quality. In the context of the present application, the terms "contrast agent" and "contrast medium" are used interchangeably.

The success of a MRI-guided interventional or intraoperative procedure generally depends on the ability of the MRI technique to provide accurate real time visualisation of the instruments and devices inserted into the patient's body. Generally most invasive devices are either metal based or made of polymeric materials, which is due to mechanical demands. Guide wires for example need to be stiff enough to enable them to be pushed past contortions and turns and to rotate as a rigid body all the way to the tip when the operator pushes and twists the other end, which is often a meter away from the tip of the wire. Hence guide wires are usually made of a steel core wound with a Teflon coated steel wire. However, when the device is metallic and/or conductive, it will act as a shield to the RF (radio frequency) irradiation and prevent it from reaching the contrast medium or agent within the device, thereby causing imaging defects in the region surrounding it. On the other hand, devices made of polymeric materials will not be visible in the MR image. It has been suggested to use non-conductive devices marked by incorporating bands of paramagnetic material such as for example dysprosium oxide. Such marker bands produce artefacts in the image. It has further been proposed to fill the instruments or devices with a paramagnetic contrast agent (e.g. GdDTPA) or a "blood pool" contrast agent, for example a ferromagnetic, ferrimagnetic or superparamagnetic contrast agent. The signal strength from such conventional MR contrast agents may however be inadequate for adequate "real time" tracking of the instrument. Furthermore, due to the toxicity of such agents, only limited amounts of agents may be administered or released into a living body. Another problem with this technology or, in fact, any technology that relies on proton imaging, is the massive background signal arising from water in the tissues and requiring either time consuming 3D data acquisition or dynamic slice positioning.

US-A-5,211,166 proposes visualisation of a part of an operative instrument by providing the instrument with a compound comprising a NMR active nucleus in, or in proximity to, the instrument. The NMR active nucleus can be any nucleus suitable for NMR, such as the NMR active isotopes of hydrogen, phosphorus, carbon, fluorine and nitrogen. Additionally, a paramagnetic relaxant is supplied and the signals of the NMR active nuclei are amplified by dynamic nuclear polarisation (DNP) to improve the visibility of the instrument. However, it is difficult using *in vivo* polarisation enhancement as suggested in this document, at normal imager field strengths (>0.2 T), to achieve sufficient polarisation due to the limited penetration of the RF pulses through tissues and the instrument material, and thereby achieve a sufficient visualisation of the instrument. Another problem that occurs at higher fields is increased heating of the tissue surrounding the device. Furthermore, the amount of paramagnetic relaxant that can be released into living body tissue is also a constraint of this method due to potential toxicity of such materials.

WO-A-02/088766 proposes a method of monitoring the position of a medical instrument *in vivo* by introducing a hyperpolarised gas into a region within or adjacent to the instrument and imaging the hyperpolarised gas using NMR. The hyperpolarised gas can be dissolved in synthetic plasma, however only to a limited extent. ¹²⁹Xe and ³He gases are specifically mentioned. Because of their low spin density, the MR sensitivity of hyperpolarised gases is significantly lower than that of a nucleus in a soluble molecule in a suitable solvent, given that other factors such as polarisation and magnetogyric ratio are similar.

Moreover, this document discloses the possibility of imaging the hyperpolarised gas within the catheter itself. Therefore, said document discloses the use of a hyperpolarised solution of a high T1 agent which has a T1 value of at least 5 seconds at a field strength of 0.001-5 T and a temperature of 20-40 °C for the manufacture of an MR contrast medium wherein a catheter is inserted into a human or non-human animal body and an MR image of at least part of said body containing said catheter is generated to visualise said catheter and wherein the use of said MR contrast medium facilitates the visualisation of said catheter.

Furthermore, WO-A-99/35508 discloses the use of a hyperpolarised solid high T1 agent or a solution thereof wherein the high T1 agent is enriched by ¹³C at one or more carbonyl or quaternary carbons and has a T1 value of at least 5 seconds at a field strength of 0.001-5 T and a temperature of 20-40 °C for the manufacture of an MR contrast medium wherein an invasive device is inserted into a human or non-human animal body.

### Summary of the invention

Hence there was a need to provide improved uses of a hyperpolarised solid high T1 agent or a solution thereof for facilitating passive visualisation of invasive devices in MR imaging, surgery and therapy. Related methods known from the prior art have drawbacks in that they expose the body under examination to high amounts of radiation, that distortions and artefacts in the MR image are unavoidable, that the contrast agents employed fail to provide the desired contrast or that they may have unacceptable toxicity at relevant doses.

It has now surprisingly been found that passive visualisation of invasive devices can be facilitated by employing a hyperpolarised solution of a high T1 agent, i.e. an agent having a T1 value of at least 5 seconds at a field strength in the range of 0.001-5 T and a temperature in the range of 20-40 °C during the time course of the visualisation procedure. The invention provides the use of a hyperpolarised solid high T1 agent or a solution thereof wherein the high T1 agent is enriched by ¹³C at one or more carbonyl or quaternary carbons and has a T1 value of at least 5 seconds at a field strength of 0.001-5 T and a temperature of 20-40 °C for the manufacture of a MR contrast medium for use in a method as defined in anyone of claims 1, 3, 5, 6 and 9 wherein an invasive device is inserted into a human or non-human animal body, preferably into the tissue and/or vasculature, and an MR image of at least a part of said body containing said device is generated to visualise said device.

Further aspects of the invention will be evident from the claims and the specification.

The non-human animal body in the context of the present application can be a mammalian, avian or reptilian body. "T1" means the longitudinal relaxation time normally measured in seconds, and "T" means Tesla.

### Detailed description of the invention

Contrast media for use in the invention comprise a hyperpolarised solid high T1 agent or a solution thereof wherein the high T1 agent is enriched by ¹³C at one or more carbonyl or quaternary carbons, i.e. an agent having a T1 value of at least 5 seconds at a field strength of 0.001-5 T and at a temperature of 20-40 °C. Such agents are well known, e.g. from WO-A-93/35508.

Preferred high T1 agents are biocompatible compounds with low toxicity. They may be used in solid form, e.g. as dispersions in a suitable solvent, but solutions of high T1 agents are preferably used, preferably in a biotolerable solvent. Particularly preferred high T1 agents are water-soluble molecules with a molecular weight below 200D (Dalton). The contrast media for use in the invention comprise high T1 agents being ¹³C enriched at one or more carbonyl or quaternary carbons. In a further preferred embodiment, these enriched high T1 agents are also deuterium labelled. Preferred ¹³C enriched high T1 agents comprise one or more ¹³C nuclei which is/are surrounded by one or more non-MR active nuclei such as O, S, C or a double bond. Preferred compounds are compounds that occur naturally in the body such as amino acids, peptides, nucleic acids, carbohydrates and intermediates or metabolites occurring in the body's normal metabolic cycles and approved pharmaceuticals.

High T1 agents for use in ablation procedures include carboxylic acids and alcohols such as ethanol enriched with ¹³C. Alternatively, ablation procedures can be performed with normal carboxylic acid and alcohol such as ethanol with a minor amount of polarised compound, i.e. a ¹³C-labelled compound, added as a marker. For use in methods of therapy, suitable therapeutics enriched with ¹³C may be used as high T1 agents, for example F-uracil and receptor targeting drugs.

Contrast media for use in the methods according to the invention preferably comprise hyperpolarised solids or solutions of high T1 agents, enriched with the mentioned ¹³C nuclei and having a T1 relaxation time of 10 seconds or more, preferably 30 seconds or more, especially preferably more than 60 seconds and even more preferably more than 100 seconds at a field strength of 0.001-5 T and at a temperature of 20 to 40 °C.

Methods for producing hyperpolarised high T1 agents are known, e.g. from WO-A-99/35508 and WO-A-99/24080. Preferred methods comprise the Dynamic Nuclear Polarisation (DNP) method and the Parahydrogen (PHIP) method. By using these methods it is possible to obtain hyperpolarised high T1 agents having a T1 of 5-seconds or more.

"Invasive devices" mean any invasive device and instrument used in interventional procedures such as but not limited to catheters including balloon catheters, balloons, optical fibres, guide wires, needles e.g. biopsy needles, electrodes, electrode leads, implants and stents.

If it is desired to shield the contrast medium from RF (radio frequency) irradiation until the device filled with the contrast medium reaches the region of interest (ROI), it has been found that carbon fibre-containing material such as carbon fibre composite material can be employed. For instance using a catheter comprising carbon fibre material will prevent depolarisation of the contrast medium by the RF irradiation employed during the placement of the catheter. The contrast medium will thus remain "invisible" inside the device until it is released at the ROI. Furthermore, carbon fibre material has low electrical conductivity compared to metals and will not cause substantial imaging artefacts during imaging or tissue heating. Invasive devices made of carbon fibre materials are preferably used in the invention.

An interventional or interoperative procedure could start with the uptake of a 2- or preferably a 3-dimensional proton image of the part of the human or non-human animal body being examined. The invasive device, e.g. a catheter, is then introduced into the vasculature, e.g. the femoral artery, or into a tissue. When it is desired to visualise the device during the procedure, the device is preferably continuously filled with contrast medium through an external duct. The signal provided by the contrast medium will allow real time visualisation and stereotactic location of the device in the 3D proton image. The proton image of the region of interest (ROI) can be updated during the procedure, e.g. to compensate for movements. It is also possible to inject doses of contrast medium into the vasculature or tissue during the introduction of the device into the body. For example, during the introduction of a catheter into an artery, it is possible to release doses of contrast medium from the device. In this way, it is for instance possible to examine the blood flow and the blood supply to an organ in real time. When the catheter has reached the region of interest, contrast medium can be released for facilitating the interventional or interoperable procedure, for example percutaneous transluminal angiography (PTCA) with a balloon catheter combined with the placement of a stent in the affected part of the artery.

The method described in the paragraph above is particularly favourable for therapeutic procedures carried out in subj ects or in parts or anatomical structures of the human or non-human animal body, that are particular sensitive to radiation. Examples of such subjects are for instance pregnant women and small children, examples of such parts or anatomical structures are for instance reproductive organs. In women, about 40% of all fertility problems result from blocked fallopian tubes. The MR contrast medium according to the invention can therefore be utilised in procedures of examining the fallopian tubes and operating them to open such blockages interventionally by inserting a catheter through the vagina. This use forms one aspect of the invention.

The invention can also be used in ablation procedures to guide the placement of clinically used devices, e.g. interstitial laser induced thermotherapy (LITT), focussed ultrasound and RF-ablation. In these uses it is of utmost importance to place the instrument used in exactly the correct position and to follow the ablation procedure, e.g. the destruction of solid tumours. By using a MR contrast medium in accordance with the present invention a catheter can be positioned exactly in the right position and the vessels and surrounding tissue, as well as the tumour or structure to be removed, can be monitored during the ablation procedure.

A further aspect of the invention involves using chemicals in the ablation procedures, either alone or together with the uses described above. When the device, e.g. a catheter, is placed in the correct position either by using a RF transparent catheter with a contrast medium or using a carbon fibre catheter, the catheter can be filled with a chemical substance effective in ablation procedures which contains, or is enriched with, hyperpolarisable nuclei. Such chemical substances are for instance carboxylic acids or alcohols. Alternatively, ablation can be performed with normal carboxylic acid or alcohol, e.g. ethanol, with a minor amount of a polarised compound, e.g. a high T1 agent, as a marker. The ablation procedure can then be monitored by MRI. For instance, ¹³C enriched ethanol can be hyperpolarised and used for this purpose.

One aspect of the invention is related to uses of the invention in therapy. As described above, a device such as a catheter can be exactly placed in the region in need of treatment and a hyperpolarisable compound, enriched with ¹³C at one or more carbonyl or quaternary carbons, can be instilled at the site. The effect of the treatment can then be followed with MRI. Examples of compounds comprise F-uracil, e.g. ¹³C enriched and receptor targeting drugs (e.g. peptides) or bactericides, fungicides etc. provided that they are ¹³C enriched at one or more carbonyl or quaternary carbons. The contrast medium will hence have a dual function as contrast medium and therapeutically active medium. Alternatively the therapeutically active compound can be a normal therapeutic compound containing a minor amount of polarised compound which works as a contrast medium.

Imaging parameters such as pulse sequences should be chosen carefully to achieve optimal results of the uses described above. Many methods for fast proton imaging are standardised on modem MRI scanners and it is obvious to one skilled in the art how to re-write the software to be suitable for other nuclei. Especially suitable methods are spiral and radial scan k-space sampling, optionally with sliding window update of the image. An additional option is the use of interleaved stereoscopic projections to allow for real-time stereoscopic visualisation by means of red-green stereo glasses or any of the many known stereo visualisation techniques.

The invention will be illustrated below by means of non-limiting examples and/or embodiments.

### Description of the Figures:

Figure 1 shows a first variant of an invasive device which does not form part of the present invention.
Figure 2 shows a carbon fibre tube as described and used in Example 2 which does not form part of.
Figure 3 shows a second variant of an invasive device which does not form part of the present invention.
Figures 4a - 4d show schematically cross-sections through further variants of invasive devices which do not form part of the present invention.
Figure 5 shows the stomach of a rat into which a guide wire has been inserted as described in Example 1.
Figure 6 shows an X-ray angiogram of a catheter placed in the left coronary artery of a pig.
Figure 7 shows a series of ¹³C MR images obtained during simultaneous retraction of a catheter form the left coronary artery of a pig and injection of a contrast medium comprising a hyperpolarised high T1 agent as described in Example 4
Figure 8 shows a series of ¹³C MR images obtained during injection of a contrast medium comprising a hyperpolarised high T1 agent through a catheter inserted into the left coronary artery of a pig as described in Example 5.
Figure 9a shows a ¹³C MR image obtained using retrospective gating during an injection of a contrast medium comprising a hyperpolarised high T1 agent through a catheter inserted into the left coronary artery of a pig as described in Example 6.
Figure 9b shows the non-hyperpolarised proton image corresponding to figure 9a.

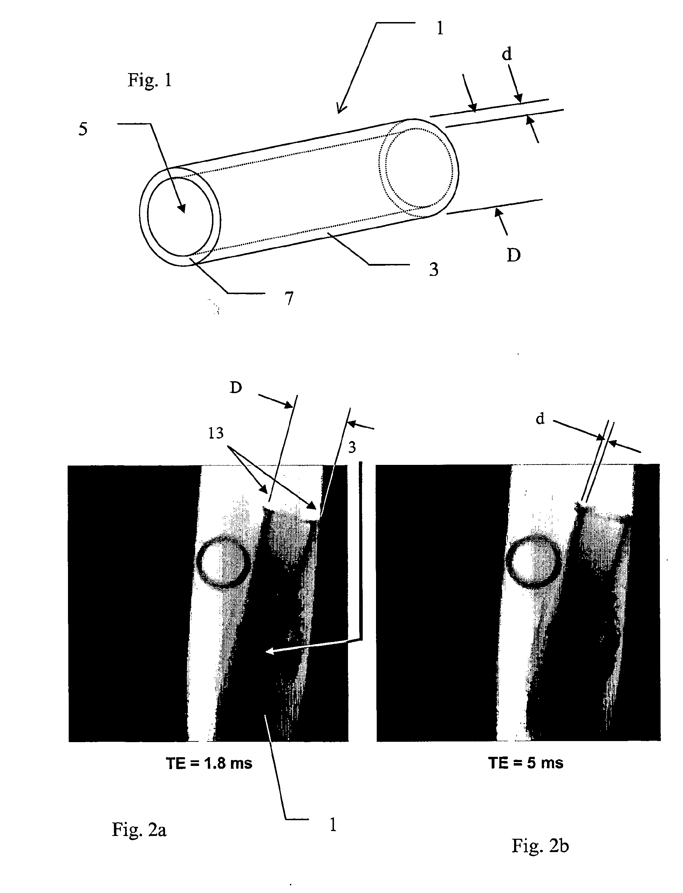

Figure 1 shows a first variant of an interventional MRI invasive device (1) for use with a contrast medium comprising a hyperpolarised high T1 agent which does not form part of the present invention. The invasive device (1) comprises a hollow, elongated body (3) of a diameter D mm. The body (3) has a central lumen (5) and a wall (7) of a thickness d mm. The invasive device (1) is intended to be inserted into the body of a human or non-human animal and thus may be made rigid or flexible depending on what it is used for, e.g. rigid if it is to be used as a needle and flexible if it is to be used as a catheter. The body (3) is made of carbon fibre containing material such as carbon fibre composite material, which has been found to be opaque to RF irradiation (as further described below). This is surprising because carbon fibre composite materials are normally considered to be insulating, as their electrical conductivites are almost 600 times less than that of copper.

In this variant, the invasive device (1) is suitable for transporting contrast medium comprising a hyperpolarised high T1 agent from a first open end, which is intended to be outside the human or non-human animal body and which is preferably connectable to a supply of contrast medium, to a second open end, which is intended to be inside or inserted into the human or non-human animal body and from which the contrast medium may be released into the ROI, for example into a blood vessel or body cavity. As the invasive device (1) is opaque to RF irradiation, the contrast medium comprising a hyperpolarised high T1 agent will not be depolarised by RF irradiation while it is shielded by the body (3).

When it is desired to visualise the devices during the entire procedure, the devices should be made of material transparent for RF (radio frequency) irradiation to provide sufficient signals from the contrast medium inside the device. Glass fibre reinforced polymer material is known to have suitable physical properties for devices such as guide wires, optical fibres, electrodes and electrode leads. Other construction polymers such as PEEK or PSU also have suitable characteristics. Needles and similar devices can be made of ceramic material such as dense zirconia, optionally coated with a polymer layer to make it less brittle or of glass fibre reinforced plastic material. Devices lacking a cavity for containing a contrast medium should be fitted with a cavity, preferably a cavity extending along the length of the device and preferably facilitating circulation of the contrast medium. Catheters may, for example, be made with double walls to allow and provide contrast medium circulation. The refill of contrast medium into the device through an external duct during the procedure will also be possible with such arrangements.

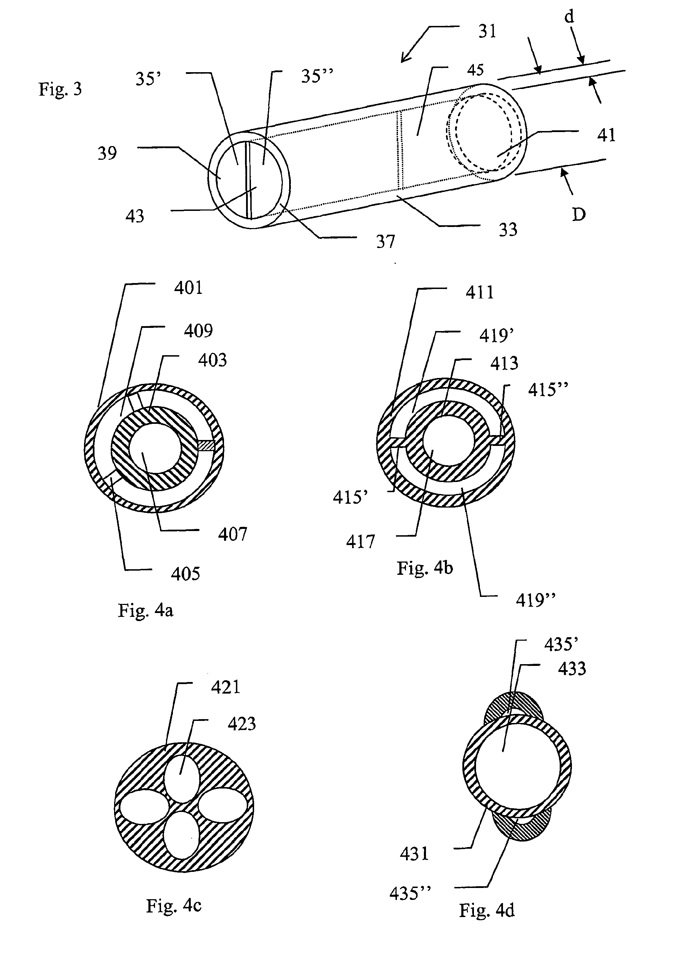

Figure 3 shows a second variant of an interventional MRI invasive device (31) for use with a contrast medium comprising a high T1 agent which does not form part of the present invention. The invasive device (31) comprises a hollow, elongated body (33) of a diameter D mm and a wall (37) of a thickness d mm. The body (33) comprises a first lumen (35') and a second lumen (35 ") which both extend from an open first end (39) of the body (33) to a closed second end (41) of the body (33). The first lumen 35' and the second lumen 35"are formed on either side of a central interior dividing wall (43) which extends from one side of the interior surface of body (33) to the, preferably diametrically, opposite side of the interior surface of body (33).

The interior dividing wall (43) does not extend all the way to the closed second end (41), thus the first (35') and second (35") lumen are in communication near the second end (41) of the device (31) through an opening (45) in the dividing wall (43). This allows a fluid, e.g. introduced into the first lumen (35') at the open first end (39) to circulate along the length of the first lumen (35), then pass through the opening (45) in the dividing wall (43) near to, or at, the closed second end (41) and return via the second lumen (35") to the open first end (39). For use in an interventional procedure, device (31) is preferably made from a material transparent to RF irradiation and a contrast medium, preferably a contrast medium comprising a hyperpolarised high T1 agent, is circulated in the invasive device (31) by being introduced though the first lumen (35') and extracted via the second lumen (35"). In such a way new contrast medium can be introduced into the device (31) as old contrast agent is extracted from it.

In a further variant which does not form part of the present invention, an invasive device comprising a hollow, elongated body is provided with a third lumen, which extends to the end of the device, which is intended to be introduced into a patient. This lumen can be used to accommodate a guide wire, which is used to position the device.

In another variant which does not form part of the present invention, an invasive device comprising a hollow, elongated body and a first and second lumen (e.g. like the device shown in figure 3) is provided with a third lumen, which extends to that end of the device that is intended to be introduced into the human or non-human body. This third lumen can be open at the end intended to be introduced into the human or non-human body and can be used to accommodate a tool. For example the third lumen could be provided with an inflatable balloon which can be inflated once the device has been manoeuvred to a suitable position inside the body. Alternatively the lumen could accommodate a biopsy needle, a stent or the like. An example of such a device can be seen in cross-section in figure 4a.

In another variant which does not form part of the present invention, an invasive device is provided with more than three lumens, of which two may be made interconnecting to allow the circulation of a fluid, e.g. a contrast medium and the others may be used for introducing tools and/or fluids into a human or non-human body. An example of such a device can be seen in cross-section in figure 4c.

### Examples

The following non-limiting examples illustrate features of the invention in as much as they fall within the scope of the claims.

### Example 1

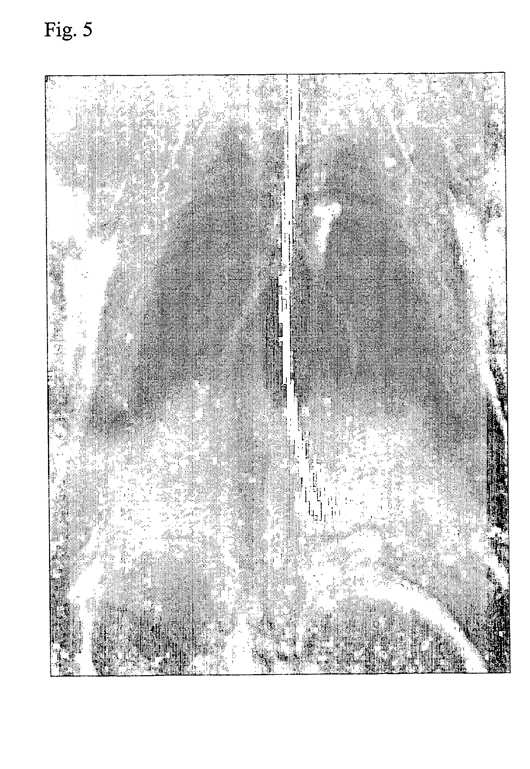

An aqueous solution of hyperpolarised dimethylsuccinate (1-¹³C) was circulated in a closed loop moulded in a guide wire of glass-fibre reinforced plastic. The T1 of this compound under these circumstances is 70 s. The guide wire was inserted in the stomach of a rat and was imaged at the ¹³C frequency at 4 images per second. The images were colour coded and overlaid with a proton image of the rat. The guide wire is clearly visible in the rat as can be seen from the image in Figure 5.

### Example 2

Figure 2 shows the results of an experiment carried out using an invasive device (1) with a body (3) made from carbon fibre with a diameter D of 10 mm and a wall (7) thickness d of 1 mm. The electrical conductivity of the carbon fibre was ~1·10⁵ S/m (c.f. copper: 5.9·10⁷ S/m). Device (1) was placed in a syringe containing water and imaged inside a 2.4 T magnet. Gradient echo images were acquired with echo time 1.8 ms as shown in Figure 2a and 5 ms as shown in Figure 2b. Similar results were obtained with both echo times. Minor susceptibility artefacts (13) can be seen near the second open end of the invasive device. No signal is obtained from the lumen (3) of the of the device (except near to the second open end where radio frequency radiation has entered the lumen (3) from said second open end) showing that the wall of the invasive device is opaque to radio frequency radiation.

### Example 3

Examples of possible variants of invasive device cross-sections are shown in figures 4a - 4d.

Figure 4a shows an invasive device formed of two concentric tubes (401, 403) with the inner tube (403) held in place in the centre of the outer tube (401) by an open system of webs (405). A first lumen (407) is formed in the interior of said inner tube and a second lumen (409) is formed by the annular space between the two tubes (401, 403).

Figure 4b shows an invasive device formed of two concentric tubes (411, 413), with the inner tube (413) held in place in the centre of the outer tube (411) by two continuous webs (415', 415 "). A first lumen (417) is formed in the interior of said inner tube (413) and a second lumen (419') and a third lumen (419") are formed by the semi-annular space between the two tubes (411, 413) and the webs (415', 415").

Figure 4c shows an invasive device comprising an elongated body (421) with four lumens (423) formed in it.

Figure 4d shows an invasive device comprising an elongated body (431) with a central lumen (433) and two crescent shaped lumens (435', 435 ") attached to the outer wall of said body (431).

Preferably the dimension D of the body of these devices is less than 20 mm, most preferably below 10 mm and greater than 1 mm, and the dimension d (wall thickness) of the body of said devices is preferably less than D/5 and most preferably less than D/10. However, said invasive devices do not form part of the invention.

### Example 4

### Preparation

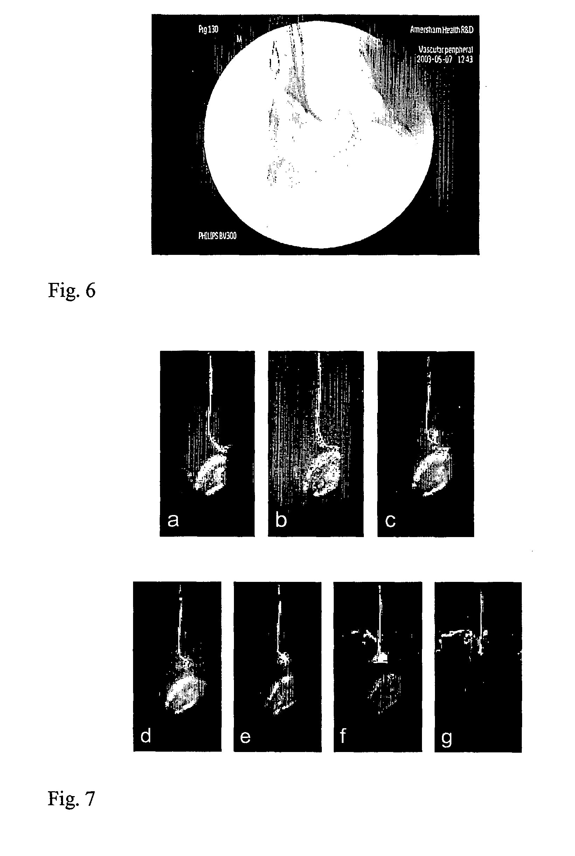

A catheter (Cobra, M, Fr.5, Terumo Europe N.V. 3001 Leuven, Belgium) was placed into the left coronary artery of a pig. Correct localisation of the catheter was confirmed by X-ray imaging using a mobile X-ray arm, a standard X-ray contrast agent was used. Figure 6 shows the X-ray angiogram obtained. An aqueous solution of hyperpolarised hydroxyethylpropionate (1-¹³C) was prepared and used as contrast medium, the polarisation was about 28%, and the concentration of hydroxyethylpropionate (1-¹³C) was about 0.5 M. For MR imaging, a 1.5 Tesla scanner (Magnetom Sonata, Siemens Medical Solutions, Erlagen, Germany) equipped with multi nuclei extension together with a transmit/receive ¹³C coil (RapidBiomedical, Würzburg, Germany) was used. In order to select projection angles for the FOV in question, standard proton scans were employed. ¹³C images were obtained using a fully balanced steady state pulse sequence.

### Conduction of the interventional procedure

¹³C images (shown in figure 7) were acquired while the catheter was retracted from the coronary artery and at the same time the contrast medium was injected through the catheter. The following imaging parameters were used: TR/TE/FA = 5.6 ms / 2.8 ms /70° and FOV / Matrix =128 x 256 mm² / 64 x 128. No gating was used and each image was generated in 350 ms. Figure 6 clearly demonstrate the ability of the method of the invention to visualize a catheter during in an interventional procedure.

### Example 5

Preparation was carried out as described in Example 4.

### Conduction of the interventional procedure

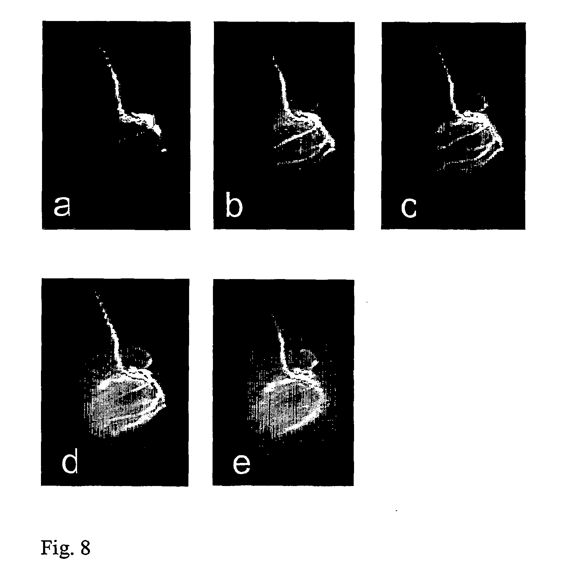

¹³C images (of which 5 extracted images are shown in figure 8) were acquired during injection of the contrast medium through the catheter. Cardiac gating was applied and one image was generated per heart cycle. The following imaging parameters were used: TR/TE/FA = 5.1 ms / 2.6 ms / 70° and FOV / Matrix = 112 x 256 mm² / 56 x 128. Each image was generated in 350 ms. Image e in figure 8 shows the myocardium, suggesting that perfusion data may also be extracted from the imaging data in addition to angiography.

### Example 6

Preparation was carried out as described in Example 4.

### Conduction of the interventional procedure

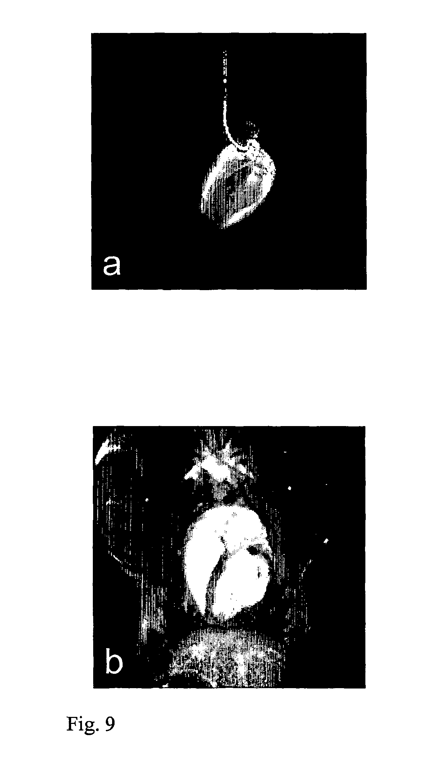

¹³C images (of which one is shown in figure 9a) were acquired during injection of the contrast medium through the catheter. Retrospective gating was applied. This method reorganizes the image data according to the simultaneously acquired cardiac gated signal. The cardiac cycle (systole-diastole) was divided into 22 phases and 16 were reconstructed. Imaging data from more than one heart beat were represented in each of the images of the series. For a spatial resolution of 2.0 x 2.0 mm² approximately 10 heart beats were needed to complete each series. The following imaging parameters were used: TR/TE/FA = 5.1 ms / 2.6 ms / 70° and FOV / Matrix = 128 x 256 mm² / 64 x 128. The total scan time was 8 s. A comparison image series was acquired using standard non-hyperpolarised proton imaging. Figure 9a shows one ¹³C image out of the series of 16 phases in the cardiac cycle. The perfusion of the cardiac muscle is evident. The corresponding non-hyperpolarised proton slice is shown in figure 9b.

## Claims

1. Use of a hyperpolarised solid high T1 agent or a solution thereof wherein the high T1 agent is enriched by ¹³C at one or more carbonyl or quaternary carbons and has a T1 value of at least 5 seconds at a field strength of 0.001-5 T and a temperature of 20-40 °C for the manufacture of a MR contrast medium for use in a method of therapy wherein an invasive device is inserted into a reproductive organ of a human or non human animal body and an MR image of at least a part of said reproductive organ containing said device is generated to visualise said device and wherein the use of said MR contrast medium facilitates the visualization of said device.

2. Use as claimed in claim 1 wherein said reproductive organ is the fallopian tubes.

3. Use of a hyperpolarised solid high T1 agent or a solution thereof wherein the high T1 agent is enriched by ¹³C at one or more carbonyl or quaternary carbons and has a T1 value of at least 5 seconds at a field strength of 0.001-5 T and a temperature of 20-40 °C for the manufacture of a MR contrast medium which contains a therapeutically active compound for use in a method of therapy wherein an invasive device is inserted into a human or non human animal body and an MR image of at least part of said body containing said device is generated to visualise said device and wherein the use of said MR contrast medium facilitates the visualization of said device.

4. Use as claimed in claim 3 wherein the effect of therapy is followed with MRI.

5. Use of a hyperpolarised solid high T1 agent or a solution thereof wherein the high T1 agent is enriched by ¹³C at one or more carbonyl or quaternary carbons, has a T1 value of at least 5 seconds at a field strength of 0.001-5 T and a temperature of 20-40 °C and is a chemical substance effective in ablation procedures for the manufacture of a MR contrast medium for use in an ablation procedure wherein a catheter is inserted into a human or non human animal body and an MR image of at least part of said body containing said catheter is generated to visualise said catheter and wherein the use of said MR contrast medium facilitates the visualization of said catheter.

6. Use of a hyperpolarised solid high T1 agent or a solution thereof wherein the high T1 agent is enriched by ¹³C at one or more carbonyl or quaternary carbons and has a T1 value of at least 5 seconds at a field strength of 0.001-5 T and a temperature of 20-40 °C for the manufacture of a MR contrast medium for use in an ablation procedure wherein a catheter is inserted into a human or non human animal body and wherein in addition a chemical substance effective in said ablation procedure is introduced through the catheter and an MR image of at least part of said body containing said catheter is generated to visualise said catheter and wherein the use of said MR contrast medium facilitates the visualization of said catheter.

7. Use as claimed in claim 5 wherein the high T1 agent is a carboxylic acid.

8. Use as claimed in claim 6 wherein the chemical substance effective in said ablation procedure is a carboxylic acid or an alcohol.

9. Use of a hyperpolarised solid high T1 agent or a solution thereof wherein the high T1 agent is enriched by ¹³C at one or more carbonyl or quaternary carbons and has a T1 value of at least 5 seconds at a field strength of 0.001-5 T and a temperature of 20-40 °C for the manufacture of a MR contrast medium for use in percutaneous transluminal angiography combined with the placement of a stent wherein a balloon catheter is inserted into a human or non human animal body and an MR image of at least part of said body containing said balloon catheter is generated to visualise said balloon catheter and wherein the use of said MR contrast medium facilitates the visualization of said balloon catheter.

## Patentansprüche

1. Verwendung eines hyperpolarisierten festen Mittels einer hohen T1 oder einer Lösung davon, wobei das Mittel einer hohen T1 angereichert ist mit ¹³C an einem oder mehreren Carbonyl- oder quarternären Kohlenstoffen und einen T1-Wert von mindestens 5 Sekunden bei einer Feldstärke von 0,001-5 T und einer Temperatur von 20-40°C besitzt, zur Herstellung eines MR-Kontrastmediums zur Verwendung in einem Therapieverfahren, wobei eine invasive Vorrichtung in ein Fortpflanzungsorgan eines menschlichen oder nicht-menschlichen tierischen Körpers eingeführt wird und ein MR-Bild von mindestens einem Teil des Fortpflanzungsorgans, das die Vorrichtung enthält, erzeugt wird, um die Vorrichtung sichtbar zu machen, und wobei die Verwendung des MR-Kontrastmediums die Sichtbarmachung der Vorrichtung vereinfacht.

2. Verwendung nach Anspruch 1, wobei das Fortpflanzungsorgan die Fallopio-Tuben sind.

3. Verwendung eines hyperpolarisierten festen Mittels einer hohen T1 oder einer Lösung davon, wobei das Mittel einer hohen T1 angereichert ist mit ¹³C an einem oder mehreren Carbonyl- oder quarternären Kohlenstoffen und einen T1-Wert von mindestens 5 Sekunden bei einer Feldstärke von 0,001-5 T und einer Temperatur von 20-40°C besitzt, zur Herstellung eines MR-Kontrastmediums, das eine therapeutisch wirksame Verbindung enthält, zur Verwendung in einem Therapieverfahren, wobei eine invasive Vorrichtung in einen menschlichen oder nicht-menschlichen tierischen Körper eingeführt wird und ein MR-Bild von mindestens einem Teil des Körpers, der die Vorrichtung enthält, erzeugt wird, um die Vorrichtung sichtbar zu machen, und wobei die Verwendung des MR-Kontrastmediums die Sichtbarmachung der Vorrichtung vereinfacht.

4. Verwendung nach Anspruch 3, wobei der Effekt der Therapie mit MRI verfolgt wird.

5. Verwendung eines hyperpolarisierten festen Mittels einer hohen T1 oder einer Lösung davon, wobei das Mittel einer hohen T1 angereichert ist mit ¹³C an einem oder mehreren Carbonyl- oder quarternären Kohlenstoffen, einen T1-Wert von mindestens 5 Sekunden bei einer Feldstärke von 0,001-5 T und einer Temperatur von 20-40°C besitzt, und eine chemische Substanz ist, die in Ablatio-Prozeduren wirksam ist, zur Herstellung eines MR-Kontrastmediums zur Verwendung in einer Ablatio-Prozedur, wobei ein Katheter in einen menschlichen oder nicht-menschlichen tierischen Körper eingeführt wird und ein MR-Bild von mindestens einem Teil des Körpers, der den Katheter enthält, erzeugt wird, um den Katheter sichtbar zu machen, und wobei die Verwendung des MR-Kontrastmediums die Sichtbarmachung des Katheters vereinfacht.

6. Verwendung eines hyperpolarisierten festen Mittels einer hohen T1 oder einer Lösung davon, wobei das Mittel einer hohen T1 angereichert ist mit ¹³C an einem oder mehreren Carbonyl- oder quarternären Kohlenstoffen und einen T1-Wert von mindestens 5 Sekunden bei einer Feldstärke von 0,001-5 T und einer Temperatur von 20-40°C besitzt, zur Herstellung eines MR-Kontrastmediums zur Verwendung in einer Ablatio-Prozedur, wobei ein Katheter in einen menschlichen oder nicht-menschlichen tierischen Körper eingeführt wird, und wobei zusätzlich eine chemische Substanz, die in der Ablatio-Prozedur wirksam ist, durch den Katheter eingeführt wird, und ein MR-Bild von mindestens einem Teil des Körpers, der den Katheter enthält, erzeugt wird, um den Katheter sichtbar zu machen, und wobei die Verwendung des MR-Kontrastmediums die Sichtbarmachung des Katheters vereinfacht.

7. Verwendung nach Anspruch 5, wobei das Mittel einer hohen T1 eine Carbonsäure ist.

8. Verwendung nach Anspruch 6, wobei die chemische Substanz, die in der Ablatio-Prozedur wirksam ist, eine Carbonsäure oder ein Alkohol ist.

9. Verwendung eines hyperpolarisierten festen Mittels einer hohen T1 oder einer Lösung davon, wobei das Mittel einer hohen T1 angereichert ist mit ¹³C an einem oder mehreren Carbonyl- oder quarternären Kohlenstoffen und einen T1-Wert von mindestens 5 Sekunden bei einer Feldstärke von 0,001-5 T und einer Temperatur von 20-40°C besitzt, zur Herstellung eines MR-Kontrastmediums zur Verwendung in einer percutanen transluminalen Angiographie kombiniert mit dem Setzen eines Stents, wobei ein Ballonkatheter in einen menschlichen oder nicht-menschlichen tierischen Körper eingeführt wird und ein MR-Bild von mindestens einem Teil des Körpers, der den Ballonkatheter enthält, erzeugt wird, um den Ballonkatheter sichtbar zu machen, und wobei die Verwendung des MR-Kontrastmediums die Sichtbarmachung des Ballonkatheters vereinfacht.

## Revendications

1. Utilisation d'un agent solide hyperpolarisé à T1 élevé ou d'une solution de celui-ci, dans laquelle l'agent à T1 élevé est enrichi en ¹³C au niveau d'un ou plusieurs carbonyles ou atomes de carbone quaternaire et présente une valeur T1 d'au moins 5 secondes sous une intensité de champ de 0,001 à 5 T et une température de 20 à 40°C afin d'assurer la fabrication d'un milieu de contraste de RM à utiliser dans un procédé de thérapie dans lequel un dispositif invasif est inséré dans un organe reproducteur d'un corps animal humain ou non humain et une image de résonance magnétique RM d'au moins une partie dudit organe reproducteur contenant ledit dispositif est produite afin de visualiser ledit dispositif et dans laquelle l'utilisation dudit milieu de contraste de RM facilite la visualisation dudit dispositif.

2. Utilisation selon la revendication 1, dans laquelle ledit organe reproductif est constitué par les trompes de Fallope.

3. Utilisation d'un agent solide hyperpolarisé à T1 élevé ou d'une solution de celui-ci, dans laquelle l'agent à T1 élevé est enrichi en ¹³C au niveau d'un ou plusieurs carbonyles ou atomes de carbone quaternaire et présente une valeur T1 d'au moins 5 secondes sous une intensité de champ de 0,001 à 5 T et une température de 20 à 40°C afin d'assurer la fabrication d'un milieu de contraste de RM qui contient un composé thérapeutiquement actif à utiliser dans un procédé de thérapie dans lequel un dispositif invasif est inséré dans un corps animal humain ou non humain et une image de résonance magnétique RM d'au moins une partie dudit corps contenant ledit dispositif est produite afin de visualiser ledit dispositif et dans laquelle l'utilisation dudit milieu de contraste de RM facilite la visualisation dudit dispositif.

4. Utilisation selon la revendication 3, dans lequel l'effet de la thérapie est suivi par IRM.

5. Utilisation d'un agent solide hyperpolarisé à T1 élevé ou d'une solution de celui-ci, dans laquelle l'agent à T1 élevé est enrichi en ¹³C au niveau d'un ou plusieurs carbonyles ou atomes de carbone quaternaire, présente une valeur T1 d'au moins 5 secondes sous une intensité de champ de 0,001 à 5 T et une température de 20 à 40°C et est une substance chimique efficace dans des procédures d'ablation afin d'assurer la fabrication d'un milieu de contraste de RM à utiliser dans une procédure d'ablation dans laquelle un cathéter est inséré dans un corps animal humain ou non humain et une image de résonance RM d'au moins une partie dudit corps contenant ledit cathéter est produite afin de visualiser ledit cathéter et dans laquelle l'utilisation dudit milieu de contraste de RM facilite la visualisation dudit cathéter.

6. Utilisation d'un agent solide hyperpolarisé à T1 élevé ou d'une solution de celui-ci, dans laquelle l'agent à T1 élevé est enrichi en ¹³C au niveau d'un ou plusieurs carbonyles ou atomes de carbone quaternaire et présente une valeur T1 d'au moins 5 secondes sous une intensité de champ de 0,001 à 5 T et une température de 20 à 40°C afin d'assurer la fabrication d'un milieu de contraste de RM à utiliser dans une procédure d'ablation dans laquelle un cathéter est inséré dans un corps animal humain ou non humain et dans laquelle, en plus, une substance chimique efficace dans ladite procédure d'ablation est introduite par l'intermédiaire du cathéter et une image de résonance RM d'au moins une partie dudit corps contenant ledit cathéter est produite afin de visualiser ledit cathéter et dans laquelle l'utilisation dudit milieu de contraste de RM facilite la visualisation dudit cathéter.

7. Utilisation selon la revendication 5, dans laquelle l'agent à T1 élevé est un acide carboxylique.

8. Utilisation selon la revendication 6, dans laquelle la substance chimique efficace dans ladite procédure d'ablation est un acide carboxylique ou un alcool.

9. Utilisation d'un agent solide hyperpolarisé à T1 élevé ou d'une solution de celui-ci, dans laquelle l'agent à T1 élevé est enrichi en ¹³C au niveau d'un ou plusieurs carbonyles ou atomes de carbone quaternaire et présente une valeur T1 d'au moins 5 secondes sous une intensité de champ de 0,001 à 5 T et une température de 20 à 40°C afin d'assurer la fabrication d'un milieu de contraste de RM à utiliser en angiographie transluminale percutanée associé à la mise en place d'un renfort dans laquelle un cathéter à ballonnet est inséré dans un corps animal humain ou non humain et une image de résonance RM d'au moins une partie dudit corps contenant ledit cathéter à ballonnet est produite afin de visualiser ledit cathéter à ballonnet et dans laquelle l'utilisation dudit milieu de contraste de RM facilite la visualisation dudit cathéter à ballonnet.
